# FASCICULE DE BREVET EUROPEEN

(11) **EP 0 770 412 B1**
(45) Date de publication et mention de la délivrance du brevet: **14.01.2004**
(21) Numéro de dépôt: 96402178.6
(22) Date de dépôt: 11.10.1996
(51) Int. Cl.: A61N 1/39

(54) **Dispositif médical du type défibrillateur/cardioverteur implantable actif à défibrillation de l'oreillette**
Medizinische Vorrichtung des Typs aktiver implantierbarer Defibrillator/Kardiovertierer für die Defibrillation des Vorhofs
Medical device of the type active implantable defibrillator/cardioverter for defibrillating the atrium

(30) Priorité: 13.10.1995 FR 9512052
(43) Date de publication de la demande: 02.05.1997
(73) Titulaire: ELA MEDICAL (Société anonyme), 92541 Montrouge (FR)
(72) Inventeur: Ripart, Alain, 91190 Gif sur Yvette (FR)
(74) Mandataire: Dupuis-Latour, Dominique

(56) Documents cités:
- EP-A- 0 060 117
- EP-A- 0 552 357
- EP-A- 0 626 182
- WO-A-92/18198
- US-A- 5 207 219
- US-A- 5 265 600
- US-A- 5 282 836

## Description

La présente invention concerne les dispositifs médicaux implantables actifs et plus particulièrement la famille des appareils chargés de délivrer au coeur des impulsions électriques de haute énergie (c'est-à-dire dépassant notablement l'énergie fournie pour la simple stimulation) en vue de mettre fin à une tachyarythmie.

Ces dispositifs sont communément appelés "défibrillateurs implantables" ou "appareils de cardioversion" (étant entendu que l'invention couvre aussi bien les défibrillateurs/cardioverteurs/stimulateurs implantables que les défibrillateurs/stimulateurs implantables).

Une classe particulière de ces appareils a pour tâche de délivrer un choc électrique au niveau de l'oreillette du patient. Ces dispositifs surveillent en permanence l'activité électrique spontanée de l'oreillette et, en cas de détection d'une fréquence anormalement élevée du rythme atrial - reconnue par l'appareil comme une fibrillation ou tachycardie auriculaire très rapide -, commande la délivrance de l'impulsion de haute énergie.

Un des problèmes connus dans l'utilisation de ces défibrillateurs de l'oreillette est le risque d'induire des fibrillations ventriculaires lors d'un choc électrique appliqué à l'oreillette. En effet le choc électrique appliqué, bien que dirigé spécifiquement par des sondes placées au voisinage des cavités hautes du coeur, peut entraîner une excitation des cellules au niveau des ventricules et déclencher une réponse ventriculaire du type d'une fibrillation, avec des conséquences dramatiques sur le pronostic vital du patient.

Pour éviter une telle conséquence, il est connu d'appliquer le choc de défibrillation auriculaire en synchronisation avec l'activité ventriculaire, et en particulier en synchronisation avec l'onde R indiquant une dépolarisation du ventricule. Les cellules sont alors toutes réfractaires et l'on évite ainsi de déclencher la défibrillation auriculaire dans la période vulnérable de l'activité ventriculaire, dénommée onde T.

Le US-A-5 207 219 décrit un procédé pour tenter de délivrer l'impulsion de défibrillation/cardioversion en synchronisme avec l'onde R. Une fois une tachycardie auriculaire détectée et la décision de défibriller prise, l'appareil mesure les intervalles entre les ondes R consécutives afin de déterminer, et ainsi prédire, la stabilité de la dépolarisation ventriculaire. Un choc sera délivré si la durée entre deux ondes R successives est supérieure à un intervalle de temps déterminé et est inférieur à une limite supérieure.

Le US-A-5 282 836 décrit un procédé où le choc de défibrillation atriale est délivré en synchronisme avec une impulsion ventriculaire, le dispositif stimulant le ventricule à une fréquence fixe avant l'épisode de défibrillation.

Toutefois ces procédés présentent l'inconvénient de ne pas s'assurer qu'une dépolarisation ventriculaire est effectivement intervenue.

Comme le font remarquer les auteurs de ces brevets, durant une fibrillation ou tachycardie auriculaire très rapide, le rythme ventriculaire et très instable, parfois très rapide, ce qui conduit à un allongement du délai R-T et à la proximité d'une onde T et de l'onde R du cycle suivant. L'attente d'une accalmie de la fréquence ventriculaire peut être longue, et le patient peut souffrir de cette attente. Par ailleurs, en cas d'absence de détection d'une dépolarisation ou dans le cas de détection de bruit qui peut leurrer le système de détection ventriculaire, il peut se produire un allongement artificiel entre deux ondes R susceptible d'entraîner une fausse analyse, et donc la délivrance non appropriée d'un choc de défibrillation atriale dans un cycle non détecté.

La présente invention a pour objet de pallier ces difficultés, en proposant un défibrillateur/cardioverteur implantable actif à défibrillation de l'oreillette qui puisse assurer la délivrance du choc en toute sécurité.

Ce dispositif est du type mentionné plus haut, tel que notamment décrit par le US-A-5 282 836 précité, c'est-à-dire qu'il comprend : des moyens de délivrance d'une énergie de défibrillation et/ou de cardioversion ; des moyens de détection de l'activité atriale qui déterminent le moment à partir duquel une énergie de défibrillation et/ou de cardioversion peut être délivrée à l'oreillette ; des moyens de détection de l'activité ventriculaire, comprenant des moyens de reconnaissance de la dépolarisation ventriculaire; des moyens de stimulation du ventricule ; et des moyens pour contrôler de manière conditionnelle les moyens de délivrance l'énergie de défibrillation et/ou de cardioversion de manière à ne délivrer l'énergie de défibrillation et/ou de cardioversion que lorsque les moyens de détection de l'activité ventriculaire indiquent la survenance d'une dépolarisation ventriculaire.

Le dispositif selon l'invention est défini dans la revendication 1.

Très avantageusement, les moyens de détection de l'activité ventriculaire comprenant de préférence en outre des moyens de mémorisation de la fréquence ventriculaire avant le moment à partir duquel un choc de défibrillation peut être délivré à l'oreillette, et les moyens de stimulation du ventricule délivrent des impulsions à une fréquence égale ou supérieure à la fréquence ainsi mémorisée.

Cette façon de faire élimine les doutes sur un éventuel phénomène de fusion entre une impulsion de stimulation et une dépolarisation spontanée du ventricule.

Selon une forme de réalisation avantageuse, les moyens de mémorisation de la fréquence ventriculaire calculent et mémorisent une moyenne glissante de la fréquence ventriculaire sur un nombre prédéterminé de cycles, notamment compris entre quatre et douze, de préférence huit.

La fréquence des impulsions délivrées par les moyens de stimulation du ventricule est avantageusement une fréquence croissante jusqu'à ce les moyens de reconnaissance indiquent la présence d'une dépolarisation ventriculaire suivant chaque impulsion de stimulation ; la fréquence de stimulation peut être limitée à une valeur prédéterminée, de préférence de 120 coups par minute.

D'autres caractéristiques et avantages de l'invention apparaîtront à la lecture de la description ci-dessous, fait en référence à l'unique dessin annexé, qui est un schéma par blocs d'un défibrillateur apte à mettre en oeuvre les enseignements de l'invention.

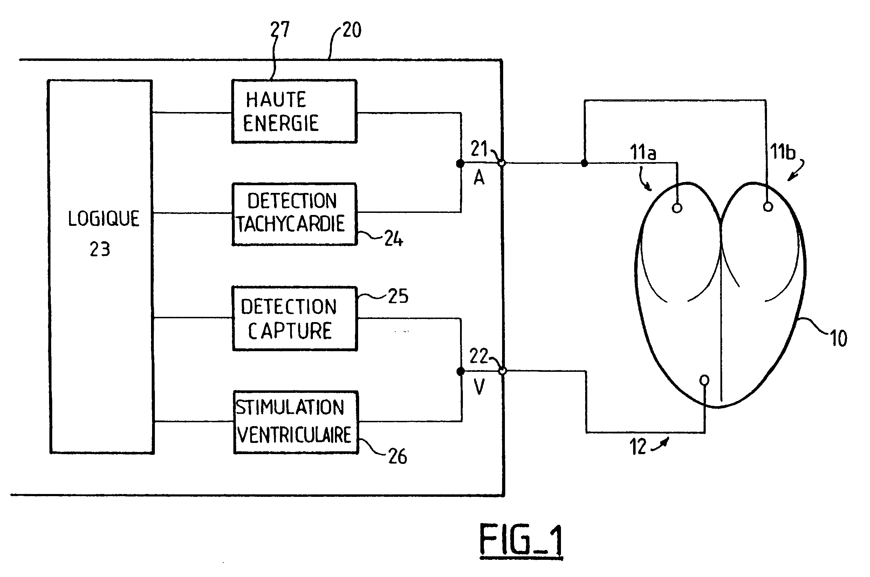

La figure 1 est un schéma par blocs d'un défibrillateur 20 qui peut être du type défibrillateur ventriculaire et atrial et composé d'une pluralité de modules interdépendants. Ses deux entrées/sorties 21 et 22 sont respectivement connectées par l'intermédiaire de sondes aux oreillettes 11a et 11b et au ventricule droit 12 du coeur 10. Ces entrées/sorties sont capables de recueillir l'activité de chacune des cavités cardiaques et de délivrer de façon connue des impulsions de stimulation et le cas échéant des chocs de défibrillation.

La présente invention peut être mise en oeuvre de façon préférentielle à l'aide des circuits et des modules qui sont employés dans les appareils du type OPUS et CHORUS fabriqués par la société ELA Médical. L'algorithme de confirmation de la capture peut être réalisé selon les enseignements du EP-A-0 552 357. Mais tout autre type de circuit ou de logique câblée ou non câblée peut être utilisée pour réaliser la présente invention.

Pour la mise en oeuvre de l'invention, l'entrée 21 est reliée à un module 24 de détection des tachycardies atriales qui, dans une forme simplifiée de l'invention, peut être constitué par un compteur de l'activité spontanée atriale qui authentifie la tachycardie lors du franchissement d'un seuil prédéterminé. Une autre méthode de détection de la tachycardie atriale est décrite dans le EP-A-0 626 182, qui fait intervenir la stabilité de l'activité auriculaire et la stabilité de l'activité ventriculaire.

L'entrée 22 est reliée à un module 25 de détection de la dépolarisation de l'activité ventriculaire. Ce module comprend des moyens de confirmation de la dépolarisation après stimulation.

Le fonctionnement d'un tel module est décrit dans le EP-A-0 552 357 précité (au nom de la Demanderesse) dont on considérera que les enseignements font partie intégrante de la présente description. Plus précisément, comme cela y est expliqué, la "capture" consiste en une phase d'étalonnage (cf. page 12 de ce document) suivie d'une phase de recherche de seuil. La phase d'étalonnage permet de déterminer les coefficients a et b d'une droite (cf. figure 4), coefficients appelés "paramètres de capture" (cf. page 9, ligne 4) ; on mesure ensuite l'amplitude du signal recueilli dans une fenêtre de 64 ms, que l'on compare à ces paramètres pour établir (cf. page 8 ligne 25) si la stimulation est efficace - auquel cas il y a "capture" - ou non.

Le défibrillateur 20 comprend par ailleurs un module de stimulation ventriculaire 26 connu en soi, et un module logique 23 qui, dans le cadre de l'invention, organise la réponse du dispositif aux différents événements qui lui sont transmis par l'intermédiaire des modules décrits précédemment. Il est en mesure de mémoriser la fréquence ventriculaire avant la survenue de la tachycardie auriculaire et de commander de façon appropriée une stimulation ventriculaire du module 26 à une fréquence supérieure à celle enregistrée, méthode par ailleurs connue sous le nom de *overdriving*. La mémorisation de la fréquence ventriculaire peut être réalisée par le calcul de la moyenne glissante du rythme ventriculaire sur un nombre prédéterminé de cycles (de quatre à seize cycles, de préférence huit).

Cette stimulation par *overdriving* peut être déclenchée en phases successives à des fréquences de plus en plus rapides jusqu'à une limite maximale de l'ordre de 120 coups par minute. Si durant une de ces phases tous les stimuli ne sont pas suivis d'une confirmation de la capture, alors la fréquence de stimulation est incrémentée jusqu'à ce que toutes les impulsions de stimulation soient suivies d'une dépolarisation du ventricule.

Ce même module 23 commande la délivrance du choc auriculaire quand les conditions fixées dans le programme qu'il contient sont remplies.

Le fonctionnement du dispositif selon l'invention peut s'expliciter de la façon suivante.

Lors du début d'une tachycardie atriale, le module 24 indique au module 23 la présence de cette tachycardie. Suivant la programmation du module 23, le dispositif décide que cette tachycardie est susceptible d'être réduite par un choc de défibrillation.

De façon concomitante à la détection de la tachycardie, le module 25 mesure l'activité du ventricule et confirme au module logique 23 la survenance d'une onde R de dépolarisation. Cette détection de la dépolarisation ventriculaire est effectuée sur des complexes électrostimulés par l'intermédiaire du module 26. Lors d'une première confirmation d'une dépolarisation ventriculaire ou lors qu'un nombre prédéfini de telles dépolarisations est confirmé, le dispositif déclenche la défibrillation auriculaire.

De cette façon on est certain que le choc de défibrillation sera donné en synchronisme avec une onde de dépolarisation ventriculaire, donc à un moment où l'on est sûr que le ventricule n'est pas vulnérable.

## Revendications

1. Dispositif médical du type défibrillateur/cardioverteur implantable actif (20) à défibrillation de l'oreillette, comprenant : des moyens (27) de délivrance d'une énergie de défibrillation et/ou de cardioversion ; des moyens (24) de détection de l'activité atriale qui déterminent le moment à partir duquel une énergie de défibrillation et/ou de cardioversion peut être délivrée à l'oreillette ; des moyens (25) de détection de l'activité ventriculaire, comprenant des moyens de reconnaissance de la dépolarisation ventriculaire après stimulation; des moyens (26) de stimulation du ventricule ; et des moyens (23) pour contrôler de manière conditionnelle les moyens (27) de délivrance l'énergie de défibrillation et/ou de cardioversion de manière à ne délivrer l'énergie de défibrillation et/ou de cardioversion que lorsque les moyens de détection de l'activité ventriculaire indiquent la survenance d'une dépolarisation ventriculaire après stimulation;
lesdits moyens de reconnaissance n'opèrent qu'en cas de délivrance préalable d'une impulsion de stimulation par les moyens (26) de stimulation du ventricule.

2. Dispositif selon la revendication 1 dans lequel les moyens (25) de détection de l'activité ventriculaire comprennent en outre des moyens de mémorisation de la fréquence ventriculaire avant le moment à partir duquel un choc de défibrillation peut être délivré à l'oreillette, et les moyens (26) de stimulation du ventricule délivrent des impulsions à une fréquence égale ou supérieure à la fréquence ainsi mémorisée.

3. Dispositif selon la revendication 2, dans lequel les moyens de mémorisation de la fréquence ventriculaire calculent et mémorisent une moyenne glissante de la fréquence ventriculaire sur un nombre prédéterminé de cycles.

4. Dispositif selon la revendication 3, dans lequel la moyenne glissante est évaluée sur un nombre de cycles compris entre quatre et douze, de préférence huit cycles.

5. Dispositif selon la revendication 2, dans lequel les moyens (26) de stimulation du ventricule délivrent des impulsions à une fréquence croissante jusqu'à ce les moyens de reconnaissance indiquent la présence d'une dépolarisation ventriculaire suivant chaque impulsion de stimulation.

6. Dispositif selon la revendication 5, dans lequel la fréquence de stimulation du ventricule est limitée à une valeur prédéterminée, de préférence de 120 coups par minute.

## Patentansprüche

1. Medizinische Vorrichtung vom Typ aktiver implantierbarer Defibrillator/Cardioverter (20) zur Herzvorhof-Defibrillation, wobei die Vorrichtung umfasst:
Mittel (27) zur Lieferung von Energie zur Defibrillation und/oder Cardioversion;
Mittel (24) zur Ermittlung der atrialen Aktivität, die den Zeitpunkt ermitteln, ab dem Energie zur Defibrillation und/oder Cardioversion am Herzvorhof zugeführt werden kann;
Mittel (25) zur Ermittlung der ventrikulären Aktivität, die Mittel zur Erkennung der ventrikulären Depolarisation nach Stimulation umfassen;
Mittel (26) zur Stimulation des Ventrikels; und
Mittel (23), um unter bestimmten Umständen die Mittel (27) zur Lieferung von Energie zur Defibrillation und/oder Cardioversion zu kontrollieren, damit nur dann Energie zur Defibrillation und/oder Cardioversion geliefert wird, wenn die Mittel zur Ermittlung der ventrikulären Aktivität das Eintreten einer ventrikulären Depolarisation nach Stimulation anzeigen;
wobei diese Mittel zur Erkennung nur im Falle einer vorausgehenden Abgabe eines Stimulationsimpulses durch die Mittel (26) zur Stimulation des Ventrikels arbeiten.

2. Vorrichtung gemäß Anspruch 1, in der die Mittel (25) zur Ermittlung der ventrikulären Aktivität außerdem Mittel zur Speicherung der Ventrikelfrequenz vor dem Zeitpunkt, ab dem ein Defibrillationsschock am Herzvorhof abgegeben werden kann umfassen, und in der die Mittel (26) zur Stimulation des Ventrikels Impulse mit einer Frequenz liefern, die gleich der so gespeicherten Frequenz oder höher ist.

3. Vorrichtung gemäß Anspruch 2, in der die Mittel zur Speicherung der Ventrikelfrequenz einen gleitenden Mittelwert der Ventrikelfrequenz über eine vorbestimmte Anzahl von Zyklen berechnen und speichern.

4. Vorrichtung gemäß Anspruch 3, in der der gleitende Mittelwert über eine Anzahl von Zyklen berechnet wird, die zwischen vier und zwölf, vorzugsweise acht Zyklen liegt.

5. Vorrichtung gemäß Anspruch 2, in der die Mittel (26) zur Stimulation des Ventrikels Impulse mit einer wachsenden Frequenz abgeben, bis die Mittel zur Ermittlung das Auftreten einer ventrikulären Depolarisation in der Folge eines jeden Stimulationsimpulses anzeigen.

6. Vorrichtung gemäß Anspruch 5, in der die Frequenz der Stimulation des Ventrikels durch einen vorbestimmten Wert begrenzt ist, vorzugsweise mit 120 Stößen pro Minute.

## Claims

1. A medical device of the active implantable defibrillator/cardioverter type (20) for defibrillating the atrium, comprising: means (27) for delivering a defibrillation and/or cardioversion energy; means (24) for detecting the atrial activity, which means determines the moment from which a defibrillation and/or cardioversion energy may be delivered to the atrium; means (25) for detecting the ventricular activity, including means for recognizing the ventricular depolarization after a stimulation; means (26) for stimulating the ventricle; and means (23) for conditionally controlling the means (27) for delivering the defibrillation and/or cardioversion energy so that the defibrillation and/or cardioversion energy be delivered only when the means for detecting the ventricular activity indicates the occurrence of a ventricular depolarization after a stimulation; said means for recognizing operating only in the case of previous delivery of a stimulation pulse by the means (26) for stimulating the ventricle.

2. The device according to claim 1, wherein the means (25) for detecting the ventricular activity further comprises means for memorizing the ventricular frequency before the moment at which a defibrillation shock be delivered to the atrium, and the means (26) for stimulating the ventricle delivers pulses at a frequency that is equal to or greater than the memorized frequency.

3. The device according to claim 2, wherein the means for memorizing the ventricular frequency calculates and memorizes a sliding average of the ventricular frequency over a predetermined number of cycles.

4. The device according to claim 3, wherein the sliding average is valuated over a number of cycles comprised between four and twelve, preferably eight, cycles.

5. The device according to claim 2, wherein the means (26) for stimulating the ventricle delivers pulses at an increasing frequency until the means for recognizing indicates the presence of a ventricular depolarization following each stimulation pulse.

6. The device according to claim 5, wherein the frequency of stimulation of the ventricle is limited to a predetermined value, preferably 120 beats per minute.
